## Europäisches Patentamt
## European Patent Office
## Office européen des brevets

(19)

(11) Veröffentlichungsnummer: **0 075 815**
**B1**

(12)

# EUROPÄISCHE PATENTSCHRIFT

(45) Veröffentlichungstag der Patentschrift:
19.03.86

(51) Int. Cl.⁴: **C 12 N 11/08,** C 12 P 1/00 //
C08F226/06

(21) Anmeldenummer: 82108643.6

(22) Anmeldetag: 18.09.82

(54) **Wasserunlösliches Proteinmaterial, dessen Herstellung und Verwendung.**

(30) Priorität: 25.09.81 DE 3138194

(43) Veröffentlichungstag der Anmeldung:
06.04.83 Patentblatt 83/14

(45) Bekanntmachung des Hinweises auf die Patenterteilung:
19.03.86 Patentblatt 86/12

(84) Benannte Vertragsstaaten:
BE CH DE FR GB IT LI NL

(56) Entgegenhaltungen:
EP - A - 0 008 100
DE - A - 2 506 085

CHEMICAL ABSTRACS, Band 67, Nr. 14, 2. Oktober
1967, Seite 6117, Nr. 64763s, Columbus, Ohio, USA
SEISHI MACHIDA et al.: "Studies on water-soluble
polymers. XI. Ionization of
poly(2-methyl-N-vinylimidazole"
CHEMICAL ABSTRACTS, Band 67, Nr. 14, 2. Oktober
1967, Seite 6118, Nr. 64764t, Columbus, Ohio, USA
SEISHI MACHIDA et al.: "Water-soluble polymers. XIII.
Copolymerization of 2-methyl-N-vinylimidazole with
itaconic acid amd methyl acrylate"
CHEMICAL ABSTRACT, Band 68, Nr. 20, 13. Mai 1968,
Seite 8466, Nr. 87619j, Columbus, Ohio, USA SEISHI
MACHIDA et al.: "Studies on water-soluble polymers.

(73) Patentinhaber: BASF Aktiengesellschaft,
Carl-Bosch-Strasse 38, D-6700 Ludwigshafen (DE)

(72) Erfinder: Goertz, Hans-Helmut, Dr., An der
Froschlache 23, D-6700 Ludwigshafen (DE)
Erfinder: Marcinowski, Stefan, Dr.,
Kopernikusstrasse 49, D-6700 Ludwigshafen (DE)
Erfinder: Sanner, Axel, Dr., Lorscher Ring 2c,
D-6710 Frankenthal (DE)

(56) Entgegenhaltungen: (Fortsetzung)
XII. Copolymerization of acrylamide with
2-methyl-1-vinylimidazole"

## Beschreibung

Die Erfindung betrifft ein wasserunlösliches Proteinmaterial, dessen Herstellung sowie die Verwendung dieses Materials zur Durchführung enzymatischer Reaktionen.

Träger zur Bindung bzw. Immobilisierung von Enzymen an Träger sind bereits bekannt (Dechema Monographie Vol.84, Verlag Chemie (1979), Seite 145 ff; ""Immobilized Enzymes" John Wiley & Sons, New York, 1978). Zur Adsorption von Enzymen wurden in der Praxis bislang Träger auf der Basis von Biopolymeren oder modifizierten Biopolymeren, wie DEAE-Cellulose oder DEAE-Sepharose, verwendet (Enzymologia 31, 214 (1969); Biotechnol. Bioeng. 9, 603 (1967)). Diese Träger besitzen jedoch nur eine geringe mechanische Stabilität und schlechte hydrodynamische Eigenschaften. Weiter ist ihre Herstellung sehr aufwendig und kompliziert. Schließlich werden sie leicht durch Mikroorganismen angegriffen und verändert.

Der Erfindung lag daher die Aufgabe zugrunde, eine Kombination aus einem Polymerisat und einem aktiven Protein zu finden, welche die oben geschilderten Nachteile weitgehend vermeidet.

Gegenstand der Erfindung ist ein wasserunlösliches, quellbares Proteinmaterial, bestehend aus einem biologisch aktiven Protein auf einem wasserlöslichen Träger, dadurch gekennzeichnet, daß es das Protein gebunden an ein wasserunlösliches, in Wasser quellbares Copolymerisat auf der Basis eines Copolymeren von N-Vinylimidazol und/oder substituierter N-Vinylimidazole und mit diesen copolymerisierbarer Monomerer enthält.

Das Copolymerisat, welches als Träger für das Proteinmaterial dient, enthält mindestens 10, vorzugsweise mindestens 30 Gew.% N-Vinylimidazol oder substituierte N-Vinylimidazole. Als substituierte N-Vinylimidazole kommen insbesondere in Betracht: 2-Methyl-, 2-Methyl-, 2-Ethyl-, 2-Propyl-, 2-Isopropyl- und 2-Phenyl-1-vinylimidazole.

Die Unlöslichkeit der Polymeren in Wasser wird insbesondere durch Zugabe von 1 bis 70, vorzugsweise 3 bis 20 Gew.% mehrfach olefinisch ungesättigter, mit den Vinylgruppen des N-Vinylimidazols bzw. der entsprechenden Derivate copolymerisierbarer Monomerer erreicht. Speziell eignen sich folgende Monomere: 2 oder mehr Vinylgruppen enthaltende Benzolderivate - vorzugsweise Divinylbenzol-, mehrfunktionelle Ester der Acryl- oder Methacrylsäurewie Butandioldiacrylat -, mehrfach ungesättigte Harnstoffderivate - wie Divinylethylenharnstoff oder Divinylpropylenharnstoff - oder Bisacrylamide - wie Methylenbisacrylamid oder Ethylenbisacrylamid.

Es ist auch möglich, weitere Monomere, die mit dem N-Vinylimidazol und Divinylbenzol copolymerisierbar sind, in einer Menge bis zu 89 Gew.% einzupolymerisieren. Sie können u.U. auch ohne Vernetzung eine für praktische Zwecke ausreichende Wasserunlöslichkeit (nur auf die Unlöslichkeit in Wasser, nicht in anderen Lösungsmitteln, kommt es an) bewirken. Solche Monomere sind z.B. Styrol, ($C_{1-4}$-Alkyl)-styrole, Acryl- und Methacrylsäure, deren Alkylester, insbesondere hydrophile Ester wie Hydroxyalkyl- oder Aminoalkylester, andere Vinylheterozyklen wie N-Vinylpyrrolidon und Vinylpyridin. Allerdings führt der Zusatz eines Comonomeren in Mengen über 20 % in der überwiegenden Zahl der Fälle zu einer Verminderung der Adsorptionseigenschaften. Auch der Ersatz des Divinylbenzols durch andere Vernetzer wie z.B. Butandioldiacrylat, Divinylethylenharnstoff oder Methylenbisacrylamid kann zu weniger guten Ergebnissen führen.

Träger der genannten Art, die zur Adsorption von Proteinen geeignet sind, können durch radikalische Polymerisation gewonnen werden. Beispielsweise kann eine Mischung der Monomeren, gegebenenfalls unter Zugabe eines nicht polymerisierbaren Zusatzstoffes, der eine Porenbildung hervorruft, mit Hilfe eines geeigneten Radikalbildners, z.B. einer Azoverbindung oder eines Peroxids, bei erhöhter Temperatur polymerisiert werden. Eine solche Polymerisation ist z.B. in der DE-OS 25 06 085 beschrieben. Wird die Polymerisation in einem Lösungsmittel durchgeführt, wobei im Falle der Vinylimidazole vor allem Wasser in Frage kommt (bei gleichzeitiger Verwendung eines wasserlöslichen Initiators), so erhält man das Polymerisat als Gel (US-PS 2 878 183). Auch eine Polymerisation in Lösung, bei der nur das Monomere, nicht jedoch das Polymere im Lösungsmittel löslich ist (Fällungspolymerisation), kommt in Betracht. Ganz besonders geeignet ist die Perl- oder Suspensionspolymerisation, da bei diesem Verfahren das Polymerisat in der für die Verwendung besonders geeigneten Perlform anfällt. Hierbei wird die Monomerenmischung, vorzugsweise zusammen mit einem organischen Zusatzstoff, unter Verwendung eines geeigneten Hilfsmittels (z.B. eines hochmolekularen Polyvinylpyrrolidons) in Wasser emulgiert und mit Hilfe eines in der Monomerenmischung gelösten Radikalbildners polymerisiert. Wegen der teilweisen Wasserlöslichkeit der Vinylimidazole empfiehlt sich ein Salzzusatz zur Wasserphase (DE-AS 19 29 501). Auch das Verfahren der umgekehrten Suspension, bei dem die wäßrige Monomerphase in einer inerten äußeren Phase, z.B. einem Kohlenwasserstoff, suspendiert ist, kann mit Erfolg angewandt werden (DE-OS 23 24 204).

Die folgenden Beispiele A bis H schildern die Herstellung geeigneter Polymerer.

## Beispiel A

Eine Mischung aus 200 Teilen 2-Methyl-1-vinylimidazol, 100 Teilen technischem Divinylbenzol (technisches Divinylbenzol bestehend aus 50 % Divinylbenzol, 50 % Ethylvinylbenzol) und 300 Teilen Butylacetat, in der 12 Teile Lauroylperoxid gelöst sind, wird in einer Lösung von 500 Teilen Na$_2$SO$_4 \cdot$ 10 H$_2$0 in 2500 Teilen Wasser, das 3 Teile Polyvinylpyrrolidon (M = 10$^6$) enthält, dispergiert und unter Rühren 8 h auf 80°C erhitzt. Das perlförmige Polymerisat wird abfiltriert, mit Wasser und Aceton gewaschen und im Vakuum getrocknet.

## Beispiel B

Eine Mischung aus 100 Teilen N-Vinylimidazol, 50 Teilen technischem Divinylbenzol und 150 Teilen Ethylacetat, in der 6 Teile Azoisobutyrodinitril gelöst sind, wird in einer Lösung von 250 Teilen Na$_2$SO$_4 \cdot$ 10 H$_2$0 in 1500 Teilen Wasser, das 3 Teile Polyvinylpyrrolidon (M = 10$^6$) enthält, dispergiert und wie in Beispiel A polymerisiert.

## Beispiel C

Eine Mischung aus 50 Teilen N-Vinylimidazol, 31 Teilen technischem Divinylbenzol und 75 Teilen Ethylacetat, die 3 Teile Azoisobutyrodinitril enthält, wird in 1500 Teilen Wasser, in dem 125 Teile Na$_2$SO$_4 \cdot$ 10 H$_2$O und 2 Teile Polyvinyl; pyrrolidon (M = 10$^6$) gelöst sind dispergiert und wie in Beispiel A polymerisiert.

## Beispiel D

Eine Mischung aus 100 Teilen 2-Methyl-1-vinylimidazol, 100 Teilen N-Vinylpyrrolidon, 100 Teilen technischem Divinylbenzol und 400 Teilen Toluol, die 10 Teile Benzoylperoxid enthält, wird in einer Lösung von 500 Teilen Natriumchlorid und 3 Teilen Polyvinylpyrrolidon (M = 10$^6$) in 2500 Teilen Wasser dispergiert und wie in Beispiel A polymerisiert.

## Beispiel E

Eine Mischung aus 45 Teilen 2-Methyl-1-vinylimidazol, 45 Teilen 2-Hydroxyethylmethacrylat, 10 Teilen technischem Divinylbenzol und 100 Teilen n-Octan, die 4 Teile Benzoylperoxid enthält, wird in einer Lösung von 200 Teilen Kochsalz und 1 Teil Polyvinylpyrrolidon (M = 10$^6$) in 750 Teilen Wasser dispergiert und wie in Beispiel A polymerisiert.

## Beispiel F

Eine Mischung aus 90 Teilen 2-Methyl-1-vinylimidazol, 10 Teilen N,N'-Divinylethylenharnstoff und 100 Teilen Ethylacetat, die 4 Teile Lauroylperoxid enthält, wird in einer Lösung von 1 Teil Xanthan und 200 Teilen Kochsalz in 750 Teilen Wasser dispergiert und wie in Beispiel A polymerisiert.

## Beispiel G

Eine Lösung von 40 Teilen N-Vinylimidazol, 20 Teilen Methylenbisacrylamid und 5 Teilen Kaliumperoxodisulfat in 40 Teilen Wasser wird unter Stickstoff 7 h auf 70°C erwärmt. Das erhaltene Gel wird zerkleinert, mit Aceton gewaschen und im Vakuum getrocknet.

## Beispiel H

Eine Mischung aus 100 Teilen N-Vinylimidazol, 50 Teilen technischem Divinylbenzol und 150 Teilen Ethylacetat, die 1 Teil Azoisobutyrodinitril enthält, wird unter Stickstoff 3 h auf 70°C erwärmt. Das erhaltene Polymerisat wird zerkleinert, mit Aceton gewaschen und im Vakuum getrocknet.

Die Polymerisate können Quellbarkeiten in Wasser von ca. 2 bis über 25 ml Feuchtvolumen pro Gramm Trockensubstanz aufweisen. Die Proteinaufnahme ist im allgemeinen bei besser quellbaren Polymerisaten höher als bei vergleichbaren weniger quellbaren, jedoch besteht kein unmittelbarer Zusammenhang zwischen Proteinaufnahme und Quellbarkeit.

Das Proteinaufnahmevermögen vinylimidazolhaltiger Polymerisate variiert je nach Zusammensetzung und Vernetzungsdichte. Es ist außerdem von Protein zu Protein verschieden. Je nach Protein kann bei der Herstellung des Polymeren die Zugabe eines inerten organischen Lösungsmittels, in dem die Monomeren löslich und die Polymeren unlöslich sind, wie z.B. Ligroin, Octan, Toluol, Essigsäureethylester oder -butylester, zur Polymerisationsmischung das Adsorptionsvermögen verbessern. Geeignete derartige Zusätze können in Stichversuchen einfach ermittelt werden. In günstigen Fällen können Adsorptionswerte von über 5 g gebundenes Protein pro g trockenes Polymerisat erreicht werden.

Die Beladung der polymeren Adsorbentien mit Protein bzw. die Entfernung eines Proteins aus einer Lösung kann entweder batchweise durch Einrühren des Polymeren in die Proteinlösung oder kontinuierlich durch überleiten der Proteinlösung über das Polymere, z.B. in einer Säule, erfolgen. Handelt es sich bei dem adsorbierten Protein um ein Enzym, so behält dieses im adsorbierten Zustand im allgemeinen zumindest einen Teil seiner ursprünglichen Aktivität. Dies erlaubt seine Verwendung als heterogener Katalysator zur Durchführung der entsprechenden Enzymreaktion. Z.B. weist das Enzym Invertase nach seiner Adsorption an einem ein Vinylimidazol enthaltenden Polymeren eine Restaktivität von ca. 20 % auf. Um die Stabilität des adsorbierten Enzyms zu erhöhen, ist eine Vernetzung des Enzyms auf dem Träger vorteilhaft. Ein hierfür geeignetes Reagenz ist z.B. Glutardialdehyd.

Mit einem biologisch aktiven Protein sind hauptsächlich Enzyme gemeint.

Enzyme, die an ein ein Vinylimidazol enthaltendes Polymeres adsorbiert werden können, sind vorzugsweise Invertase, Glucoseisomerase, Amyloglucosidase, alpha- und beta-Amylase, Aminosäureacylase, Penicillinacylase und Hydantoinase. Ferner sind geeignet: Oxidoreduktasen - wie Alkoholdehydrogenase, Lactatdehydrogenase, Aminosäureoxidase, Peroxidase, Katalase, Glucoseoxidase, Alkoholoxidase, Succinatdehydrogenase, Glutamatdehydrogenase, Uricase, Phenoloxidase, Catecholoxidase, Monoaminooxidase, Lipoxygenase, Luciferase, Nitratreduktase, Nitritreduktase, Chlorperoxidase, Acetaldehyddehydrogenase, Aldehydoxigenase, Diaphorase, Cholesterinoxidase, Glutarthioreduktase, Hydroxysteroiddehydrogenase, Xanthinoxidase, Dopaminhydroxylase, Cytochromoxidase, Monoaminooxidase, Diacetylreduktase, Superoxiddismutase, Limonatdehydrogenase -;
Transferasen - wie Polynucleotidphosphorylase, Dextransucrase, Phosphorylase, Carbamatkinase, Aminotransferase, Transaldolase, Methyltransferase, Pyruvatkinase, Carbamoyltransferase, Phosphofructokinase, Dextransynthetase -; Hydrolasen - wie Lipase, Esterase, Lactase, Lysozym, Cellulase, Urease, Trypsin, Chymotrypsin, Glutaminase, Asparaginase, Papain, Ficin, Pepsin, Leucinaminopeptidase, Carboxypeptidase A + B, Naringinase, Bromelain, Subtilisin, Phospholiphase, Isoamylase, Cephalosporinamidase, Adenosindeaminase, Penicillinase, Maltase, Dextranase, Desoxyribonuclease, Sulfatase, Pullulanase, Phosphatase, alpha-Galactosidase, Dextranase, beta-Glucanase -; Lyasen - wie Tryptophanase, Tyrosindecarboxylase, Oxinitrilase, Phenylalanindecarboxylase, Phenylalaninammoniumlase, Aminosäuredecarboxylase, Pyruvatdecarboxylase, Fumarase, Enolase, Aspartase, Aminolaevulindehydratase, Carboanhydratase -; Isomerasen - wie Aminosäureracemase, Triosephosphatisomerase -; Ligasen - wie Glutathionsynthetase.

Die erfindungsgemäßen Präparate weisen einen besonders hohen Proteingehalt auf und zeigen gute hydrodynamische Eigenschaften und eine gute mechanische Stabilität. Weiter sind sie einfach herzustellen.

Die folgenden Beispiele erläutern die Herstellung des Proteinmaterials:

**Beispiel 1**

100 mg eines nach Beispiel A hergestellten Polymerisates (Teilchengröße 250 bis 500µm) werden in 100 ml einer 0,01 %igen wäßrigen Hämoglobinlösung gerührt und die Abnahme des Hämoglobins in der Lösung photometrisch verfolgt. Nach 20 min sind 15 %, nach 60 min 47 % des Proteins am Polymeren adsorbiert.

**Beispiel 2**

200 mg eines nach Beispiel B hergestellten Polymeren (Teil-chengröße 250 bis 500µm) werden 64 h bei 4°C in 20 ml Lösung von 100 mg alpha-Amylase (130 Units/mg) in 0,005 M Natriumacetatpuffer vom pH 6,0 geschüttelt. Dann wird der Träger jeweils bei Raumtemperatur zweimal 5 min und zweimal 1 h in je 50 ml 0,05 M Natriumacetatpuffer von pH 6,0 gewaschen. Zur Ermittlung der immobilisierten enzymatischen Aktivität wird der Träger bei 30°C 30 min in 50 ml Lösung von 5 g Stärke nach Zulkowsky (Merck) in 0,016 M Natriumacetatpuffer (pH 6,0) geschüttelt. Die entstehenden Oligosaccharide werden mit 3,5-Dinitrosalicylsäure nach P. Berenfeld (Methods in Enzymology, Vol. I, 149, Academic Press, 1955), bestimmt. Aktivität des Trägers: 3,0 g hydrolysierte Stärke pro g und pro h.

**Beispiel 3**

200 mg eines nach Beispiel C hergestellten Polymeren (Teil-chengröße 250 bis 500µm) werden 64 h bei 4°C in 20 ml Lösung von 100 mg beta-Amylase (28 Units/mg) in 0,05 M Natriumacetatpuffer.(pH 4,8) geschüttelt. Dann wird der Träger jeweils bei Raumtemperatur zweimal 5 min und zweimal 1 h in je 50 ml 0,05 M Natriumacetatpuffer von pH 4,8 gewaschen. Zur Ermittlung der immobilisierten enzymatischen Aktivität wird der Träger bei 30°C 30 min in 50 ml Lösung von 5 g Stärke nach Zulkowsky (Merck) in 0,016 M Natriumacetatpuffer (pH 4,8) geschüttelt. Die gebildete Maltose wird mit 3,5-Dinitrosalicylsäure nach P. Berenfeld (loc. cit.) bestimmt. Aktivität des Trägers: 2,3 g gebildete Maltose pro g und pro h.

**Beispiel 4**

An 200 mg eines nach Beispiel B hergestellten Polymeren (Teilchengröße 250 bis 500µm) werden analog zu dem in Beispiel 2 angegebenen Verfahren Amyloglucosidase gebunden. Die Aktivität des Trägers beträgt 15,3 g gebildete Glucose pro g und h.

### Beispiel 5

25 mg eines nach Beispiel D hergestellten Polymeren werden 64 h bei 4°C mit einer Lösung von 250 mg Invertase (150 Units/mg) geschüttelt. Danach wird das Polymerisat bei Raumtemperatur zweimal 5 min und einmal 1 h in je 100 ml 0,0025 M Natriumacetatpuffer (pH 5,3) gewaschen. Das Polymere wird 10 min in 50 ml einer Lösung von 17,5 g Sucrose in 0,001 M Natriumacetatpuffer (pH 5,3) geschüttelt und der Hydrolysegrad polarimetrisch bestimmt. Aktivität des Trägers 275 g Sucrose pro g und h. Gebundene Enzymmenge: 2,4 mg pro mg Polymeres.

### Beispiel 6

Analog Beispiel C werden 25 mg eines nach Beispiel E hergestellten Polymeren mit Invertase beladen. Aktivität: 300 g Sucrose pro g Polymerisat und h. Gebundene Enzymmenge: 1,3 mg pro mg Polymerisat.

### Beispiel 7

Analog Beispiel 5 werden 25 mg eines nach Beispiel F hergestellten Polymerisates mit Invertase beladen. Aktivität: 194 g Sucrose pro g Polymerisat und h. Gebundene Enzymmenge: 1,5 mg pro mg Polymerisat.

### Beispiel 8

Analog Beispiel 5 werden 25 mg eines nach Beispiel G erhaltenen Polymeren mit Invertase beladen. Aktivität: 150 g Sucrose pro g und h.

### Beispiel 9

Analog Beispiel 5 werden 25 mg eines nach Beispiel H erhaltenen Polymeren mit Invertase beladen. Aktivität: 6 g Sucrose pro g und h.

### Beispiel 10

1 g eines nach Beispiel C hergestellten Polymeren wird analog zu Beispiel 5 mit Invertase beladen. Danach wird das Polumerisat in eine Säule gefüllt und auf 30°C thermostatisiert. Durch die Säule wird mit einer Flußgeschwindigkeit von 100 ml/h eine Lösung von 640 g Sucrose/l (auf pH 5,3 eingestellt) gepumpt. Die Hydrolyse der Sucrose wird polarimetrisch verfolgt. Nach 1 Tag beträgt der Hydrolysegrad 92 %, nach 20 Tagen 38 %.

### Beispiel 11

Der analog Beispiel 10 beladene Träger wird vor der Verwendung mit 20 ml einer 0,1 %igen Lösung von Glutardialdehyd in Wasser behandelt. Damit beträgt der Hydrolysegrad nach 1 Tag 95 %, nach 20 Tagen 85 %.

### Patentansprüche

1. Wasserunlösliches Proteinmaterial, bestehend aus einem biologisch aktiven Protein auf einem wasserunlöslichen Träger, dadurch gekennzeichnet, daß es das Protein gebunden an ein wasserunlösliches Copolymerisat auf der Basis eines Copolymeren von N-Vinylimidazol oder substituierter N-Vinylimidazole und mit diesen copolymerisierbarer Monomerer enthält.

2. Proteinmaterial gemäß Anspruch 1, dadurch gekennzeichnet, daß es als Copolymerisat ein Perlpolymerisat enthält.

3. Proteinmaterial gemäß Anspruch 1 oder 2, dadurch gekennzeichnet, daß der Träger als copolymerisierbares Monomeres eine mehrfach olefinisch ungesättigte Komponente einpolymerisiert enthält.

4. Proteinmaterial gemäß Anspruch 3, dadurch gekennzeichnet, daß die mehrfach ungesättigte Komponente des Trägers Divinylstyrol, ein mehrfunktioneller Ester der Acryl- oder Methacrylsäure, ein mehrfach olefinisch ungesattigtes Harnstoffderivat oder ein ebensolches Säureamid ist.

5. Proteinmaterial gemäß Anspruch 4, dadurch gekennzeichnet, daß der Träger als weiteres Comonomeres Styrol, ein $(C_{1-4}$-Alkyl)-styrol, Acryl; oder Methacrylsäure, einen Alkyl- oder Aminoalkylester derselben oder einen Vinylheterocyclus einpolymerisiert enthält

6. Verfahren zur Herstellung des Proteinmaterials gemäß-Ansprüchen 1 bis 5, dadurch gekennzeichnet, daß man das Copolymerisat mit einer wäßrigen Lösung oder Suspension eines biologisch aktiven Proteins in Kontakt bringt.

7. Verwendung des Proteinmaterials gemäß den Ansprüchen 1 bis 5 zur Durchführung enzymatischer Reaktionen.

## Claims

1. A water-insoluble protein material consisting of a biologically active protein on a water-insoluble carrier, wherein the protein is bonded to a water-insoluble copolymer based on N-vinylimidazole or substituted N-vinylimidazoles and monomers which are copolymerizable therewith.

2. A protein material as claimed in claim 1, wherein the copolymer is a bead polymer.

3. A protein material as claimed in claim 1 or 2, wherein the carrier contains, as the copolymerizable monomer, a polyolefinically unsaturated component.

4. A protein material as claimed in claim 3, wherein the polyunsaturated component of the carrier is divinylstyrene, a polyfunctional ester of acrylic acid or methacrylic acid, a polyolefinically unsaturated urea derivative or a polyolefinically unsaturated acid amide.

5. A protein material as claimed in claim 4, wherein the carrier contains, as a further comonomer, styrene, a ($C_{1-4}$-alkyl)-styrene, acrylic acid or methacrylic acid, an alkyl or aminoalkyl ester thereof or a vinylheterocyclic compound.

6. A process for the preparation of a protein material as claimed in claims 1 to 5, wherein the copolymer is brought into contact with an aqueous solution or suspension of a biologically active protein.

7. The use of a protein material as claimed in claims 1 to 5 for carrying out enzymatic reactions.

## Revendications

1.- Matiere protéique insoluble dans l'eau constituée d'une protéine biologiquement active sur un support insoluble dans l'eau, caractérisée par le fait qu'elle contient la protéine liée à un copolymérisat, insoluble dans l'eau, à base d'un copolymère de N-vinylimidazole ou de N-vinylimidazole substituée et de monomère copolymérisable avec ceux-ci.

2.- Matière protéique selon la revendication 1, caractérisée par le fait qu'elle contient, comme copolymérisat, un polymérisat en suspension.

3.- Matière protéique selon la revendication 1 ou 2, caractérisée par le fait que le support contient en polymerisation, comme monomère copolymérisable, un composant à multiple insaturation oléfinique.

4.- Matière protéique selon la revendication 3, caracterisée par le fait que le composant,à multiple insaturation, du support est le divinylstyrène, un ester plurifonctionnel de l'acide acrylique ou méthacrylique, un dérivé d'urée à multiple insaturation oléfinique ou un amide de même genre.

5.- Matiére protéique selon la revendication 4, caractérisée par le fait que le support contient, en polymérisation, comme autre comonomère, du styréne, un alkyle en $C_{1-4}$- styrène, l'acide acrylique ou méthacrylique, un ester alkylique ou aminoalkylique de celui-ci ou un hétérocycle vinylique.

6.- Procédé de préparation de matière protéique selon les revendications 1 à 5, caractérisé par le fait que l'on met en contact le copolymérisat avec une solution aqueuse ou une suspension d'une protéine biologiquement active.

7.- Utilisation de la matière protéique selon les revendications 1 à 5 pour la mise en oeuvre de réactions enzymatiques.